# EUROPÄISCHE PATENTSCHRIFT

(11) **EP 2 013 156 B1**
(45) Veröffentlichungstag und Bekanntmachung des Hinweises auf die Patenterteilung: **01.09.2010**
(21) Anmeldenummer: 07728517.9
(22) Anmeldetag: 25.04.2007
(51) Int. Cl.: C05D 9/00, C11C 3/10, C07C 67/03, C07C 69/24, C07C 69/52, C07C 69/533, C01F 7/06, C11D 1/00

(54) **VERFAHREN ZUR UMESTERUNG VON ESTERN**
METHOD FOR TRANSESTERIFICATION OF ESTERS
PROCÉDÉ DE TRANSESTÉRIFICATION D'ESTERS

(30) Priorität: 28.04.2006 DE 102006019883
(43) Veröffentlichungstag der Anmeldung: 14.01.2009
(73) Patentinhaber: Krause-Röhm-Systeme AG, 80714 München (DE)
(72) Erfinder: KRAUSE, Eberhard, 16540 Hohen Neuendorf (DE); RÖHM, Valentin, 80714 München (DE)
(74) Vertreter: Hofstetter, Alfons J.
(86) Internationale Anmeldenummer: PCT/EP2007/054062
(87) Internationale Veröffentlichungsnummer: WO 2007/125069

(56) Entgegenhaltungen:
- EP-A2- 0 093 339
- WO-A-2005/026080
- WO-A-2005/063954
- GB-A- 634 411

## Beschreibung

### Technisches Gebiet

Die Erfindung betrifft ein Verfahren zur Umesterung mindestens einer, mindestens eine Estergruppe umfassenden Komponente mit mindestens einer, mindestens eine Hydroxygruppe umfassenden Komponente.

### Stand der Technik

Bei der Aluminium-Produktion gemäß dem Bayer-Verfahren wird mit Hilfe von Natronlauge aus fein gemahlenem Bauxit Al₂O₃ herausgelöst. Aus der dabei erhaltenen Natriumaluminat-Lösung wird nach Animpfen mit Kristallisationskeimen reines Al(OH)₃ (Gibbsit) ausgefällt, aus welchem nach weiteren Verfahrensschritten elektrolytisch metallisches Aluminium gewonnen wird. Zurück bleibt ein Gemisch, das sich chemisch betrachtet hauptsächlich aus Eisen-IIIoxiden bzw. -hydroxiden, Titanoxiden, Aluminiumoxidresten, Quarzsand, Kalziumoxid, Natriumoxid und Restnatronlauge zusammensetzt. Aufgrund seiner durch Eisen-III-oxid hervorgerufenen roten Farbe wird dieser Rückstand als Rotschlamm oder "red mud" bezeichnet.

Zu jeder produzierten Tonne Aluminium fallen je nach Qualität des verwendeten Bauxits 1 bis 1,5 Tonnen Rotschlamm als nicht vermeidbarer Begleiter an. Die dabei jedes Jahr entstehende Menge beträgt mehrere Millionen Tonnen und stellt zusammen mit den schon vorhandenen Rotschlamm-Abfällen ein ernsthaftes Umwelt- und Entsorgungsproblem dar. Das Hauptproblem ist die aufgrund seines Natronlaugengehalts hohe Alkalität des Rotschlamms mit pH-Werten zwischen 11 und 13. Darüber hinaus stellen toxisch wirkende Aluminium-Ionen zusammen mit Eisen-Verbindungen eine große Gefahr für das Grundwasser dar und erschweren eine umweltgerechte Lagerung zusätzlich. Die Entsorgung des Rotschlamms erfolgt zurzeit im Wesentlichen durch Einlagerung in abgedichteten Deponien. Die am Boden der Deponie austretende Natronlauge wird gesammelt und in den Bayer-Prozess rückgeführt. Diese Form der Lagerung ist jedoch teuer und aufwändig, da große Deponieflächen und -anlagen benötigt werden und hohe Kosten für den Transport des Rotschlamms anfallen. Zudem sind die durch die Deponierung entstehenden Langzeitkosten nur schwer kalkulierbar und stellen ein zusätzliches wirtschaftliches Problem dar.

Zahlreiche Versuche wurden unternommen, um den bislang als Abfallprodukt betrachteten Rotschlamm in nutzbare Wertstoffe umzuwandeln und einer wirtschaftlichen Verwertung zuzuführen. EP-A2-0 093 339 offenbart die Verwendung von Rotschlamm bei der hydrierenden Verflüssigung von Kohle. Jeder sinnvolle Ansatz muss dabei in erster Linie auf eine Verringerung des stark alkalischen pH-Wertes abzielen, sollte aber in zweiter Linie auch das im Rotschlamm enthaltene Potenzial so weit wie möglich ausschöpfen und eine umfassende Verwertung der enthaltenen Komponenten bieten. Die Verarbeitung wird dadurch erschwert, dass die Partikel des Rotschlamms bedingt durch den Herstellungsprozess einen im Schnitt sehr geringen Durchmesser im Bereich zwischen 0,1 µm und 1 µm besitzen.

Ein neueres Verfahren von Virotec International LTD, geschützt als "Basecon^{™} Technology", erreicht durch den Umsatz von Rotschlamm mit Meerwasser eine Verringerung des pH-Wertes auf etwa 9 und eröffnet dadurch verschiedene Anwendungsmöglichkeiten für den dealkalisierten Rotschlamm, wie etwa den Einsatz als Flockungsmittel oder als Behandlungsmittel für saure Abwässer bzw. saure Böden.

Nachteilig an diesem Verfahren ist der Umstand anzusehen, dass die Verwendung von jährlich etwa 1 Millionen Tonnen im Rahmen dieses Verfahrens weniger als 2 % der Jahresproduktion entspricht und es daher nicht geeignet ist, die jährlich anfallende Rotschlamm-Menge zu bewältigen und insbesondere keine Lösung für die bereits deponierten Rotschlamm-Abfälle darstellt. Weiterhin ist als nachteilig anzusehen, dass kein umfassender Gebrauch der verschiedenen, im Rotschlamm enthaltenen Wertstoffe erfolgt und so das vorhandene wirtschaftliche und ökologische Potenzial nicht genutzt wird.

Aufgabe der vorliegenden Erfindung ist es daher, ein Verfahren zu schaffen, welches eine möglichst umfassende stoffliche Verwertung des bereits deponierten sowie des jährlich neu anfallenden Rotschlamms ermöglicht.

### Darstellung der Erfindung

Die Aufgabe wird erfindungsgemäß durch ein Verfahren zur Umesterung eines Esters mit einem Alkohol unter Verwendung von Rotschlamm als reaktionsfördernder Komponente mit den Merkmalen des Patentanspruchs 1 gelöst.

Vorteilhafte Ausgestaltungen mit zweckmäßigen und nicht-trivialen Weiterbildungen der Erfindung sind in den weiteren Patentansprüchen beschrieben.

Erfindungsgemäß wird Rotschlamm in einem Verfahren zur Umesterung einer oder mehrerer, wenigstens eine Estergruppe umfassender Verbindungen mit einer oder mehreren Verbindungen, welche mindestens eine Hydroxygruppe umfassen, als reaktionsfördernde Komponente eingesetzt. Ein solches Verfahren bietet verschiedenste Vorteile. Rotschlamm enthält neben großen Mengen Natronlauge auch verschiedene Metalloxide und -hydroxide und dient so als stark basische und katalytisch wirksame Reaktionskomponente, welche die alkoholischen Verbindungen in Alkoholate überführt und die Umesterungsreaktion ermöglicht. Neben dieser basischen Esteralkoholyse laufen aufgrund des im Rotschlamm gebundenen Restwassers gleichzeitig auch basische Esterhydrolysen ab, in denen die Estergruppen enthaltenden Komponenten in die entsprechenden Carbonsäuren und Alkohole aufgespalten werden. Bei dieser Reaktion werden Hydroxid-lonen verbraucht, so dass sich der pH-Wert des Rotschlamms verringert. Dass im Lauf der Reaktion alkalische Bestandteile verbraucht werden, würde unter normalen Bedingungen einen klaren Nachteil darstellen, da entweder die alkalischen Bestandteile ständig ersetzt oder auf eine Wiederverwendung des Katalysators verzichtet werden müsste. Bei dem erfindungsgemäßen Verfahren werden diese Gegebenheiten jedoch in vorteilhafter Weise genutzt, da Rotschlamm in praktisch unbegrenzter Menge verfügbar ist und die im Rotschlamm enthaltenen alkalischen Bestandteile keinen Kostenfaktor, sondern im Gegenteil den Kern des Problems darstellen und in möglichst großer Menge verbraucht werden sollen. Das erfindungsgemäße Verfahren liefert daher in einem Schritt umgeesterte Produkte, freie alkoholische Verbindungen, Salze freier Carbonsäuren und dealkalisierten Rotschlamm, die sich in der Regel sehr einfach voneinander trennen lassen. Auf diese Weise ermöglicht das erfindungsgemäße Verfahren eine umfassende stoffliche Verwertung von Rotschlamm unter Gewinnung verschiedener Wertstoffe, wobei sowohl bereits deponierte als auch neu anfallende Rotschlämme problemlos eingesetzt und in großen Mengen verwertet werden können.

In einer vorteilhaften Ausgestaltung der Erfindung umfasst das Verfahren folgende Schritte: a) Vermischen der mindestens eine Estergruppe umfassenden Komponente, der mindestens eine Hydroxygruppe umfassenden Komponente und dem Rotschlamm, b) Erwärmen und/oder Durchmischen des erzeugten Gemischs für die Dauer eines vorbestimmten Zeitintervalls und c) Abtrennen wenigstens einer ersten flüssigen Phase von wenigstens einer zweiten Phase, wobei die flüssige Phase wenigstens einen Carbonsäurealkylester und die zweite Phase wenigstens dealkalisierten Rotschlamm umfasst. In vielen Fällen werden Umesterungen bereits durch Mischen der Reaktanden gestartet. Oft verlaufen diese Reaktionen aber so langsam, dass ein Erwärmen des Reaktionsgemisches notwendig wird. Daher werden die eine Estergruppe umfassende Komponente, die alkoholische Komponente und der Rotschlamm gemischt und im Anschluss unter Rühren für eine bestimmte Zeit erwärmt. Auf diese Weise kann der Reaktion die notwendige Aktivierungsenergie zugeführt und ein möglichst rascher und vollständiger Umsatz der Edukte bei weitgehender Neutralisierung des Rotschlamms erzielt werden. Nach Beendigung der Reaktion trennen sich die einzelnen Reaktionsprodukte in mindestens zwei verschiedenen Phasen auf und können voneinander separiert werden. Eine erste flüssige Phase enthält dabei mindestens einen Carbonsäureester als Produkt der Umesterung. Der dealkalisierte Rotschlamm sedimentiert in den meisten Fällen problemlos innerhalb kurzer Zeit und bildet eine feste Phase, in welcher auch Salze der freien Carbonsäuren vorliegen können. Je nach Edukt- und Produkt-Zusammensetzung und Reaktionsführung kann der Rotschlamm statt als fester Niederschlag aufgrund seiner geringen Partikelgröße auch als polymorphe, kolloidale Suspension vorliegen, die aber eine klare Phasengrenzlinie zur ersten flüssigen Phase aufweist und durch ihre intensive rote Färbung einfach identifizierbar ist.

In einer weiteren vorteilhaften Ausgestaltung des erfindungsgemäßen Verfahrens ist die mindestens eine Estergruppe umfassende Komponente ein Pflanzenöl. Pflanzenöle bestehen überwiegend aus Mono-, Di- und Triglyceriden und sind in großer Vielfalt und in großen Mengen weltweit erhältlich. Neben verschiedenen anderen denkbaren Pflanzenölen eignen sich insbesondere Palmöle, Sojaöle oder Rapsöle als Edukt für das erfindungsgemäße Verfahren, da sie wirtschaftlich gesehen preisgünstige und global verfügbare Edukte darstellen und aufgrund ihrer biologischen Abbaubarkeit unter Umweltgesichtspunkten als weitgehend unproblematisch zu bewerten sind. Das deutsche Umweltbundesamt stuft Pflanzenöle beispielsweise in die Wassergefährdungsklasse 1 und damit als lediglich schwach wassergefährdend ein. Da es im Rahmen des erfindungsgemäßen Verfahrens nicht erforderlich ist, hochreine Öle einzusetzen, lassen sich durch die Verwendung von grob gepressten oder verunreinigten Pflanzenölen zusätzliche Kosten einsparen. Es können auf diese Weise sogar Abfallprodukte der Pflanzenölindustrie einer vorteilhaften Verwertung zugeführt und ebenfalls zur Wertstoffgewinnung herangezogen werden. Pflanzenöle besitzen darüber hinaus einen gewissen Anteil an freien Fettsäuren, mit denen bereits ein Teil der alkalischen Rotschlammbestandteile neutralisierbar ist. Dabei entstehen die entsprechenden Salze der Fettsäuren, bei denen es sich letztlich um Seifen handelt. Diese können erfindungsgemäß als zusätzlicher Wertstoff abgetrennt und für verschiedene Zwecke verwendet werden. Ein ebenfalls während dieses Verfahrens entstehender Wertstoff ist Glycerin, das durch die basische Esterhydrolyse aus den im Pflanzenöl enthaltenen Glyceriden freigesetzt wird. Glycerin findet beispielsweise in der Pharma- und Kosmetikindustrie Verwendung als wertvoller Grund- und Rohstoff und kann durch das erfindungsgemäße Verfahren in großer Menge gewonnen werden. Alle Haupt- und Nebenprodukte der Reaktion können somit beim Einsatz von Pflanzenölen als Edukt des erfindungsgemäßen Verfahrens weiterverwendet werden und stellen selbst einen Wertstoff dar.

In einer weiteren vorteilhaften Ausgestaltung des erfindungsgemäßen Verfahrens umfasst die mindestens eine Hydroxygruppe umfassende Komponente der Umesterung einen oder mehrere Alkohole aus der Gruppe Methanol, Ethanol, Propanol und Butanol. Kombiniert mit der Verwendung von Pflanzenöl als Estergruppen umfassender Komponente, lassen sich auf besonders einfache Weise Fettsäurealkylester gewinnen, die als so genannter Biodiesel den derzeit umweltverträglichsten Kraftstoff für Dieselfahrzeuge darstellen. Die Vorteile des Produkts Biodiesel sind dem Fachmann bekannt. Biodiesel weist deutlich geringere Abgaswerte auf als fossiler Kraftstoff, ist nahezu schwefelfrei, ungiftig, CO₂-neutral und biologisch abbaubar. Zusätzlich wird der Rußausstoß eines mit Biodiesel betankten Kraftwagens halbiert.

Eine einfache und kostengünstige Reaktionsführung wird bei dem erfindungsgemäßen Verfahren dadurch erzielt, dass entweder die Estergruppe umfassende Komponente oder die Hydroxygruppe umfassende Komponente selbst als Lösungsmittel fungiert und zur Lösung bzw. Aufschlämmung der übrigen Komponenten verwendet wird.

In einer weiteren vorteilhaften Ausgestaltung des erfindungsgemäßen Verfahrens wird im ersten Verfahrensschritt a) zunächst der Rotschlamm in der die Hydroxygruppe umfassenden Komponente aufgeschlämmt und anschließend die die Estergruppe umfassende Komponente zugemischt. Dadurch kann ein Verkleben des Rotschlamms verhindert und eine möglichst homogene Mischung erzeugt werden, die Voraussetzung für eine schnelle Umsetzung der Reaktanden ist.

In einer weiteren vorteilhaften Ausgestaltung des erfindungsgemäßen Verfahrens wird die Temperatur wenigstens einer Komponente vor ihrer Zumischung auf einen vorbestimmten Temperaturwert eingestellt. Es hat es sich dabei als vorteilhaft gezeigt, insbesondere die die Estergruppe umfassende Komponente vor ihrer Beimischung vorzuwärmen. Dies kann beispielsweise durch die Verwendung eines Rohrwärmetauschers erfolgen und stellt eine einfache Methode dar, einerseits die Reaktion zu aktivieren und andererseits während des Verfahrens anfallende Wärmeenergie in den Prozess energie- und kostensparend zurückzuführen.

In einer weiteren vorteilhaften Ausgestaltung des erfindungsgemäßen Verfahrens wird das Reaktionsgemisch in Schritt b) vorzugsweise unter Rühren für die Dauer einer Stunde auf etwa 60°C erwärmt. In den meisten Fällen wird so ein optimaler Kompromiss zwischen Wirtschaftlichkeit, Reaktionsdauer und Ausbeutemaximierung erreicht.

In einer weiteren vorteilhaften Ausgestaltung des erfindungsgemäßen Verfahrens wird nach Schritt a) und insbesondere während Schritt b) eine vierte Komponente aufgefangen, die wenigsten eine bei Raumtemperatur und Normaldruck gasförmige Komponente umfasst. In Schritt b) aufgefangene gasförmige Verbindungen lassen sich vorteilhaft in vielfältiger Weise weiternutzen. Eine mögliche Nutzung besteht dabei in der thermischen Verwertung von Reaktionsgasen und der Rückführung der dabei gewonnenen thermischen Energie in den Prozess, beispielsweise zum Vorwärmen der Edukte oder zum Erwärmen des Reaktionsgemisches.

In einer weiteren vorteilhaften Ausgestaltung des erfindungsgemäßen Verfahrens wird nach Schritt b) in einem zusätzlichen Schritt d) eine fünfte und eine sechse Komponente aus dem Gemisch abgetrennt, wobei die fünfte Komponente wenigstens eine Verbindung aus der Gruppe der C₁- bis C₄-Monoalkohole und die sechste Komponente wenigstens Wasser umfasst. In Abhängigkeit der eingesetzten Edukte und der gewählten Prozessführung hat es sich als vorteilhaft erwiesen, nach Ablauf der Reaktion die entstandenen bzw. nicht abreagierten oder als Lösungsmittel eingesetzten Alkohole aus dem Reaktionsgemisch zu entfernen. Dabei wird vorzugsweise ebenfalls vorhandenes Restwasser mit entfernt. Auf diese Weise kann einerseits eine Rückgewinnung von Wertstoffen erzielt und andererseits die anschließende, in Schritt c) durchzuführende Abtrennung der wenigstens zwei Phasen erleichtert und beschleunigt werden.

In einer weiteren vorteilhaften Ausgestaltung des erfindungsgemäßen Verfahrens umfasst der zusätzliche Schritt d) dabei eine Variation von Temperatur- und/oder Druckbedingungen. Eine einfache Möglichkeit stellt dabei das Erwärmen des Reaktionsgemisches über den Siedepunkt der zu entfernenden Verbindungen mit anschließender Abdestillierung der fünften und sechsten Komponente dar. Sollen beispielsweise nur die Alkohole Methanol oder Ethanol zusammen mit Restwasser entfernt werden, genügt eine Erhöhung der Temperatur des Reaktionsgemisches auf etwa 98°C unter Normaldruckbedingungen.

In einer weiteren vorteilhaften Ausgestaltung des erfindungsgemäßen Verfahrens umfasst die Variation der Temperaturbedingungen eine Regulierung des Temperaturwerts auf etwa 80°C und die Variation der Druckbedingungen eine Regulierung des Druckwerts auf einen gegenüber dem Normaldruck stark verminderten Wert, insbesondere unter 250 hPa. Diese Variationen der Temperatur- und Druckbedingungen führen zu einer deutlichen Beschleunigung des Destillationsprozesses. Diese Variationen sind insbesondere dann vorteilhaft, wenn die fünfte Komponente auch längerkettige Alkohole wie Propanol oder Butanol umfasst, da diese Siedepunkte von 97°C bzw. 118°C besitzen. Die Variationen sind aber nicht auf die Anwendung bei diesen Verbindungen beschränkt und sind ebenso denkbar zur Entfernung von Wasser, Methanol oder Ethanol. Die Variation der Druckbedingungen kann vorzugsweise mit Hilfe von handelsüblichen Membranpumpen erreicht werden. Geeignete Parameterwerte beispielsweise für das Abdestillieren eines Ethanol-Wasser Gemisches sind eine Temperatur von etwa 80°C und ein Druck unterhalb von 250 hPa.

In einer weiteren vorteilhaften Ausgestaltung des erfindungsgemäßen Verfahrens umfasst das Verfahren nach Schritt d) einen zusätzlichen Schritt e), in welchem die in Schritt d) gemeinsam abgetrennte fünfte und sechste Komponente aufgetrennt wird. Wird beispielsweise in Schritt d) ein Ethanol-Wasser Gemisch gemeinsam abgetrennt, hat es sich als vorteilhaft gezeigt, das abdestillierte Ethanol-Wasser Gemisch im Sinne einer nachhaltigen Rohstoffnutzung wieder in seine Einzelbestandteile aufzutrennen und den Alkohol beispielsweise für einen weiteren Verfahrensdurchlauf zu verwenden. Denkbar ist jedoch auch eine Anwendung von Schritt e) auf sämtliche abtrennbaren Gemische wie beispielsweise Methanol-Wasser oder Propanol-Wasser.

In einer weiteren vorteilhaften Ausgestaltung des erfindungsgemäßen Verfahrens umfasst Schritt e) wenigstens ein Verfahren zur Trennung homogener Gemische, insbesondere ein Extraktivdestillationsverfahren. Da Alkohol und Wasser oft homogene, azeotrope Gemische bilden, die sich durch einfache Rektifikation nicht vollständig trennen lassen, ist für diesen Fall die Verwendung eines geeigneten Trennverfahrens vorgesehen. Dabei bieten sich neben Extraktivdestillationsverfahren auch alle sonstigen geeigneten Trennverfahren wie beispielsweise Membrantrennverfahren an. Beim Extraktivdestillationsverfahren wird dem azeotrop siedenden binären Gemisch eine dritte Verbindung als Schleppmittel beigemischt, wodurch ein ternäres Gemisch erzeugt wird. Als Schleppmittel bietet sich in idealer Weise Glycerin an, welches zum Beispiel bei Verwendung von Pflanzenölen als Umesterungsedukt ein Produkt der Reaktion darstellt und daher in großer Menge verfügbar ist. Ebenfalls denkbar als Schleppmittel sind aber auch weitere geeignete Verbindungen wie Paraffinöl oder Ethandiol.

Eine weitere vorteilhafte Weiterbildung der Erfindung ergibt sich, wenn Schritt c) wenigstens ein Destillations- und/oder Filterungs- und/oder Sedimentations- und/oder Dekantierverfahren umfasst. Die Abtrennung der ersten flüssigen Phase von der zweiten, den Rotschlamm enthaltenden Phase durch einen Dekantierschritt zieht in vorteilhafter Weise Nutzen aus den unterschiedlichen Dichtewerten der einzelnen Reaktionsprodukte. So lassen sich auf einfache und kostengünstige Weise die einzelnen Komponenten des Reaktionsgemisches praktisch quantitativ voneinander trennen. Ebenso zur Auftrennung des Gemischs geeignet sind Sedimentations- und Filterungsverfahren. Denkbar ist auch eine Auftrennung mit Hilfe von Destillationsverfahren, um in vorteilhafter Weise flüchtige von nicht-flüchtigen Komponenten, insbesondere dem Rotschlamm, abzutrennen.

In einer weiteren vorteilhaften Ausgestaltung des erfindungsgemäßen Verfahrens umfasst wenigstens ein Sedimentationsverfahren ein Abtrennen von Rotschlammsedimenten.

Eine weitere vorteilhafte Ausgestaltung des erfindungsgemäßen Verfahrens umfasst wenigstens ein Filterungsverfahren, wobei der Filterungsschritt mittels Rotschlamm-Sedimenten erfolgt. Durch ein Hintereinanderschalten von Sedimentation und Filtration können die im ersten Sedimentationsschritt erhaltenen Rotschlammsedimente im folgenden Filterungsschritt vorteilhafterweise die aktive Filterkomponente bilden. Aufgrund der geringen Partikelgröße des Rotschlamms erhält man so auf besonders einfache und kostensenkende Weise ein extrem leistungsstarkes Filterelement mit einer hohen Rückhaltewirkung für feste Komponenten. Denkbar ist auch, zunächst anstelle eines Sedimentationsschrittes mit einem groben Filterelement Rotschlamm abzufiltrieren und in weiteren Filterungsschritten als Filterelement zu verwenden.

In einer weiteren vorteilhaften Ausgestaltung des erfindungsgemäßen Verfahrens umfasst wenigstens ein Filterungsverfahren einen Filterungsschritt mittels eines Vakuumfilters. Da es in einem solchen Fall nicht notwendig ist, einen zusätzlichen Sedimentationsschritt vor der Filtrierung auszuführen, ergibt sich so eine deutliche Zeitersparnis und eine damit verbundene Kostensenkung aufgrund des höheren Durchsatzes an Reaktionsgemisch.

In einer weiteren vorteilhaften Ausgestaltung des erfindungsgemäßen Verfahrens ist der Vakuumfilter mehrstufig ausgebildet. Aufgrund der geringen Partikelgröße des Rotschlamms hat es sich als vorteilhaft gezeigt, einen mehrstufigen Vakuumfilter zur Vermeidung des Zusetzens eines einzelnen Filterelements zu verwenden.

In einer weiteren vorteilhaften Ausgestaltung des erfindungsgemäßen Verfahrens wird zumindest ein Teil eines durch den Filterungsschritt erzeugten Filterkuchens thermisch verwertet und insbesondere verbrannt. Die Hauptkomponente des Filterkuchens besteht aus Rotschlamm, welcher aufgrund seiner großen Oberfläche verschiedene brennbare Reaktionsprodukte bindet. Verwendet man beispielsweise Pflanzenöl als Estergruppe umfassende Komponente des Verfahrens, so enthält der Rotschlamm-Filterkuchen nach Abschluss der Reaktion neben Resten der alkoholischen Komponente Glycerin, Seifen und Carbonsäureester als brennbare Bestandteile.

In einer weiteren vorteilhaften Ausgestaltung des erfindungsgemäßen Verfahrens werden dem Filterkuchen vor dem thermischen Verwerten Kunststoff- und/oder Holz- und/oder biologische Abfälle zugemischt. Neben dem Zumischen von brennbaren Abfallprodukten wie Kunststoff-, Holz- oder biologischen Abfälle ist auch die Zumischung von Stroh-, Zellstoff- oder brennbaren organischen Abfällen denkbar. Auch dieser Schritt eröffnet so die Möglichkeit, als Abfall eingestuftes Material einer vorteilhaften Weiterverwendung zugänglich zu machen.

In einer weiteren vorteilhaften Ausgestaltung des erfindungsgemäßen Verfahrens wird die bei der thermischen Verwertung entstehende Wärmeenergie wenigstens einem der Verfahrensschritte a) bis c) und/oder gegebenenfalls d) und/oder e) zurückgeführt. Die thermische Energie kann beispielsweise zum Vorwärmen eines Edukts der Reaktion vor dessen Zumischung dienen, zur Erwärmung des Reaktionsgemisches auf den vorbestimmten Temperaturwert oder zur Durchführung eines an die Reaktion anschließenden Trennschritts, insbesondere eines Destillationsschritts. Alternativ ist auch eine Nutzung der thermischen Energie zur Stromerzeugung denkbar.

In einer weiteren vorteilhaften Ausgestaltung des erfindungsgemäßen Verfahrens umfasst das Verfahren nach Schritt c) einen zusätzlichen Schritt f), welcher ein Abtrennen mindestens einer, mindestens Glycerin umfassenden Komponente umfasst. Verwendet man beispielsweise Pflanzenöle als Estergruppe umfassende Komponente als Edukt in Schritt a) des Verfahrens, so erhält man unter anderem Glycerin als Reaktionsprodukt der Umesterung. Da Glycerin selbst ein wichtiger Wertstoff ist und in vielfacher Weise weiterverwendet werden kann, wird es daher in vorteilhafter Weise in Schritt f) vom übrigen Gemisch abgetrennt.

In einer weiteren vorteilhaften Ausgestaltung des erfindungsgemäßen Verfahrens umfasst das Verfahren nach Schritt f) einen weiteren Schritt g), in welchem die mindestens eine, mindestens Glycerin umfassende Komponente in wenigstens zwei Qualitätsstufen aufgetrennt wird. Dies ist insbesondere dann vorteilhaft, wenn das abgetrennte Glycerin nicht die für eine direkte Weiterverwendung erforderliche Reinheit aufweist. Durch Schritt g) wird daher sichergestellt, dass die Anforderungen der jeweiligen Weiterverwendungszwecke entsprechende Berücksichtigung im Verarbeitungsprozess finden.

In einer weiteren vorteilhaften Ausgestaltung des erfindungsgemäßen Verfahrens umfasst das Verfahren nach Schritt c) einen weiteren Schritt h), welcher das Abtrennen mindestens eines Natriumionen enthaltenden Salzes mindestens einer Carbonsäure aus wenigstens einer abgetrennten Phase umfasst. Entstehen während der Umesterung durch basische Esterhydrolyse Natriumionen enthaltende Salze von Carbonsäuren, ist in vorteilhafter Weise vorgesehen, diese, ebenfalls Wertstoffe darstellenden Verbindungen im zusätzlichen Verfahrensschritt h) abzutrennen. Wird beispielsweise Pflanzenöl als Estergruppe umfassende Komponente des Verfahrens eingesetzt, stellen die so erhaltenen Carbonsäuresalze die Salze von Fettsäuren dar und sind somit als Seifen zu bezeichnen.

Ein weiterer Vorteil des erfindungsgemäßen Verfahrens besteht darin, dass aus bislang als Abfall aufgefassten Produkten Wertstoffe gewonnen werden können und sogar die während der Reaktion entstehenden, normalerweise unerwünschten Nebenprodukte ihrerseits Wertstoffe darstellen. Diese zusätzlich entstehenden Wertstoffe können in besonders vorteilhafter Weise für verschiedene Verwendungszwecke eingesetzt werden.

Eine weitere vorteilhafte Ausgestaltung der Erfindung umfasst die Verwendung des in Schritt h) abgetrennten, mindestens Natriumionen enthaltenden Salzes mindestens einer Carbonsäure als Pflanzenbehandlungsmittel, insbesondere bei Schädlingsbefall. Die Verwendung dieses Carbonsäuresalzes als Schädlingsbekämpfungsmittel ist nicht nur wirtschaftlich vorteilhaft, da es die Nutzung eines Reaktionsproduktes des erfindungsgemäßen Verfahrens ermöglicht, sondern auch ökologisch, da die Carbonsäuresalze biologisch abbaubar und somit unter Umweltaspekten weitgehend unbedenklich sind.

In einer weiteren vorteilhaften Ausgestaltung der Erfindung wird das als Pflanzenbehandlungsmittel verwendete Natriumionen enthaltende Salz mindestens einer Carbonsäure mit mindestens einem Lösungsmittel, insbesondere Wasser verdünnt. Dadurch kann die gewünschte Zielkonzentration einfach und situationsabhängig ohne Einschränkung der Wirkung oder der biologischen Verträglichkeit eingestellt werden. Das Carbonsäuresalz lässt sich so als wässrige Lösung mit Hilfe gängiger landwirtschaftlicher Geräte leicht auf das zu behandelnde Pflanzenmaterial aufbringen.

Eine weitere vorteilhafte Ausgestaltung der Erfindung umfasst die Verwendung des in Schritt h) abgetrennten, mindestens Natriumionen enthaltenden Salzes mindestens einer Carbonsäure als Detergens, insbesondere in Reinigungs- und/oder Waschmitteln. Die Verwendung des abgetrennten Carbonsäuresalzes als Detergens wird in vorteilhafter Weise durch den seifenartigen Charakter dieser Verbindungsklasse begünstigt und ermöglicht eine zusätzliche wirtschaftliche Verwertung eines Produktes des erfindungsgemäßen Verfahrens.

Der durch das Verfahren entstehende dealkalisierte Rotschlamm besitzt seinerseits eine große Bandbreite an möglichen Verwendungszwecken.

Eine weitere vorteilhafte Ausgestaltung der Erfindung umfasst die Verwendung des abgetrennten, dealkalisierten Rotschlamms als Eisen liefernder Bestandteil eines insbesondere in der Landwirtschaft verwendbaren Eisendüngers. Die ausreichende Versorgung von Pflanzen mit Eisen ist insbesondere in der Landwirtschaft von großer Bedeutung, da Eisen beispielsweise bei Gehölzen und Rasen die Blattgrünbildung und dadurch das Wachstum fördert. Der Rotschlamm kann dabei je nach Einsatzzweck in seiner neutralen Form verwendet oder gezielt auf einen leicht basischen pH-Wert eingestellt werden. Dies ist insbesondere im Hinblick auf den zunehmenden sauren Regen vorteilhaft, da so eine Regulierung auf den natürlichen pH-Wert des Bodens erzielt wird.

Eine weitere vorteilhafte Ausgestaltung der Erfindung sieht vor, dass der Eisendünger zusätzlich mindestens Kalkstein umfasst. Das zusätzliche Beimischen von Kalkstein trägt in vorteilhafter Weise den Anforderungen des landwirtschaftlichen Pflanzenanbaus Rechnung und vereint verschiedene pflanzenwachstumsfördernde Eigenschaften in einem Produkt, da eine geregelte Kalkversorgung die Grundlage jeder Düngung darstellt. Zusätzlich sorgt der beigemischte Kalk für eine Regulierung des pH-Wertes des Eisendüngers.

Weitere Vorteile, Merkmale und Einzelheiten der Erfindung ergeben sich anhand der folgenden Beschreibungen mehrerer Ausführungsbeispiele.

### Beispiel 1:

Zur möglichst vollständigen Neutralisierung von alkalischem Rotschlamm bei gleichzeitiger Gewinnung möglichst vieler Wertstoffe, werden bei einem ersten Ausführungsbeispiel für das Umesterungsverfahren 400 ml Rapsöl als Estergruppen umfassende Komponente, 100 ml Ethanol (96 %) als Hydroxygruppen umfassende Komponente und 400 g Rotschlamm als reaktionsfördernde Komponente eingesetzt.

Zunächst werden bei Raumtemperatur Ethanol und Rotschlamm in einem Reaktionsbehälter vermischt und homogenisiert. Die homogenisierte Mischung wird mit Rapsöl versetzt und unter Rühren auf 60°C erwärmt. Ein Anspringen der Reaktion macht sich dabei oft durch ein Entstehen gasförmiger Verbindungen bemerkbar. Nach einer Reaktionszeit von etwa einer Stunde wird die Temperatur bis zum Sieden der Mischung auf etwa 100°C erhöht, um überschüssiges Ethanol und Wasser aus dem Reaktionsgemisch zu entfernen. Das abdestillierte Ethanol-Wasser Gemisch wird aufgefangen und kann mit dem Fachmann geläufigen Methoden in seine Einzelkomponenten aufgetrennt werden. Das verbleibende Produktgemisch wird zunächst grob durch einen Glasfaserfilter filtriert. Der hauptsächlich Rotschlamm enthaltende Filterkuchen wird anschließend selbst als Filterkörper verwendet und besitzt aufgrund der geringen Partikelgröße eine hervorragende Trennleistung. Das Filtrat wird zwei weitere Male mit Hilfe des Rotschlammfilters filtriert bis im Filtrat keine Rotfärbung mehr erkennbar ist. Das trübe Filtrat besitzt jetzt einen neutralen pH-Wert und wird in einen Scheidetrichter überführt. Innerhalb von etwa 30 Minuten bis 2 Stunden erfolgt eine Aufteilung des Gemischs in drei Phasen, welche in einfacher Weise durch Dekantieren voneinander abgetrennt werden. Die erste abgetrennte Phase mit einer Dichte von 1,22 g/cm³ enthält dabei als Wertstoff Glycerin, die zweite Phase besteht aus einer Suspension verschiedener Fettsäuresalze und die dritte Phase enthält als Biodiesel verwendbaren Rapsethylester mit einer Dichte von 0,87 g/cm³.

### Beispiel 2:

In einem an sich bekannten Vormischer mit einem Volumen von 1 m³ werden 0,5 t Rotschlamm mit einem Wasseranteil von etwa 30 % (w/w, entsprechend ca. 150 I) und 500 I Methanol (ca. 90 %) aufgerührt. An Stelle von Methanol kann alternativ auch Ethanol, ein weiterer Alkohol oder ein Gemisch unterschiedlicher Alkohole verwendet werden. Anschließend wird das Alkohol-/Wasser-Gemisch abgezogen. Der Wassergehalt im Rotschlamm verringert sich dadurch auf ca. 10% (w/w), wodurch sich die weitere Bearbeitung der Rotschlammmasse deutlich verbessert. Das stark alkalische Alkohol-/Wasser-Gemisch wird durch Vakuum-Flüssigextraktion in 96 % Alkohol und Wasser aufgetrennt. Das stark alkalische Wasser wird anschließend in einer Gaswäsche-Vorrichtung eingesetzt, die einem Dampferzeuger nachgeschaltet ist.

In einem zweiten Arbeitsgang wird anschließend Bioalkohol mit 96 % Ethanol in einer Menge von 250 l, entsprechend dem Zweieinhalbfachen der für die Umesterung benötigten Menge, zugegeben und für einige Minuten durchgerührt. Anschließend wird die flüssige Masse in einen Umesterungsreaktor mit ca. 1,5 m³ Volumen umgepumpt und ungefiltertes Rapsöl aus der Presse in einem Volumen von ca. 1 m³ eingespeist. Das Rapsöl wird zuvor auf 60-70°C vorgewärmt, was vorzugsweise mit Hilfe von Wärmerückgewinnung aus einer der nachfolgenden Reaktionsstufen erfolgt.

Die Mischung wird anschließend unter Rühren auf Temperaturen größer 50°C, vorteilhafterweise auf mindestens 60°C erwärmt und ca. eine Stunde bei dieser Temperatur gehalten. Durch Kontrolle des pH-Wertes der Lösung kann die Beendigung des Prozesses in einfacher Weise festgestellt und gegebenenfalls zusätzliches Pflanzenöl bzw. zusätzliches Alkohol-/Rotschlamm-Gemisch nachgeführt werden, um einen nahezu vollständigen Umsatz zu erreichen. Anschließend wird die Temperatur auf ca. 80°C erhöht und das Gemisch aus verbliebenem Restwasser und bei der Umesterung nicht verbrauchtem Alkohol unter vermindertem Druck bei ca. 250 hPa abgesaugt. Zur Druckverringerung kann beispielsweise eine Membran-Vakuumpumpe verwendet werden. Über eine nachgeschaltete Flüssigextraktionstufe erfolgt anschließend wieder eine Trennung in Wasser und Alkohol. Der Alkohol wird rückgeführt, das Wasser ist neutral und kann als normales Brauchwasser genutzt werden.

Die verbleibende Ester-/Glyzerin-/Seife-Mischung wird über mehrere Sedimentfilter abgepumpt, gefiltert und dann in einen Dekanter gepumpt, wobei sich rasch aufgrund der Dichteunterschiede eine Separierung in 3 Phasen ergibt, die jeweils getrennt abgepumpt und als einzelnes Produkt verwendet werden können. Das alkalifreie, neutrale Rotschlammsediment enthält vergleichsweise viel brennbare Restlösung und wird thermisch verwertet, gegebenenfalls unter Zufügung von Holzabfällen oder Ähnlichem. Der nach der Oxidation verbleibende Mineralrest kann als Eisendünger verwertet oder weiter in Magnetit und eine Zementzuschlagkomponente aufgespaltet und getrennt verwertet werden.

### Beispiel 3:

Rotschlamm aus einer Deponie mit einem Wassergehalt zwischen 24-27 % (w/w) wird zunächst mit Restwärme aus einer späteren Verbrennungsstufe in einem Vakuumreaktor bei einem Grobvakuum von etwa 100 mbar auf ca. 60°C erhitzt, wodurch Wasser durch Vakuumdestillation entzogen wird wodurch ein Großteil des Wassers entzogen wird. Nach ca. 30 min sind nur noch einige Prozent Wasser enthalten. Der getrocknete Rotschlamm wird dann in einen weiteren Reaktor abgesaugt.

Im nächsten Schritt wird Methanol in einem Verhältnis Methanol:Rotschlamm von etwa 1:2 (v/w) zugegeben und das Gemisch intensiv durchmischt, um das Methanol an alle Mineralkörner-Oberflächen zu bringen und die Bildung von Natrium-Methanolat durch Reaktion mit an den Oberflächen chemisorbierter NaOH zu fördern. Die Reaktionstemperatur liegt dabei etwa zwischen 40-50°C, die Reaktionsdauer beträgt üblicherweise zwischen 10-15 min.

Anschließend wird vorzugsweise mit Abwärme des Prozesses auf eine Temperatur zwischen 50-70°C vorgeheiztes Pflanzenöl, beispielsweise Raps-, Soja- oder Palmöl, im einem Verhältnis zwischen 1:1 und 1:2 (v/w) bezogen auf Rotschlamm in den Reaktor zugegeben. Die Mischungstemperatur wird bei 60-65°C gehalten und die Reaktion für etwa 30 min durchgeführt, wobei wegen der extrem hohen Oberfläche des Rotschlamms kürzere Reaktionszeiten in der Regel nicht sinnvoll sind. Während der Reaktion wird das Gemisch intensiv durchmischt. Dabei finden mehrere Prozesse statt:
Die Hauptreaktion besteht dabei in der Umesterung des Pflanzenöls mit der Bildung von Methylestern der freien bzw. aus dem Pflanzenöl freigesetzten Fettsäuren.

Die untergeordneten Nebenreaktionen umfassen im Wesentlichen Verseifungsreaktionen zwischen freier Natronlauge und durch Esterspaltung gebildeten oder im Pflanzenöl bereits enthaltenen freien Fettsäuren unter Bildung von Seife und Glyzerin in Gegenwart von Wasser, die Hydrolyse von Proteinen, Phospholipiden u. a. durch die stark alkalische Lösung zu Beginn des Prozesses sowie die Neutralisierung der Natronlauge bzw. des Natriumcarbonats unter Bildung von Natriumsalzen der Fettsäuren.

Nach Beendigung der Umesterung wird aus einem Vorratsbehälter ein unpolares Lösungsmittel - beispielsweise Hexan - in einer dem Pflanzenöl entsprechenden Menge in den Reaktor gepumpt. Anschließend werden unter intensivem Durchmischen alle unpolaren bzw. überwiegend unpolaren Bestandteile extrahiert, welche hauptsächlich nicht abreagiertes Pflanzenöl sowie die entstandenen Methylester umfassen.

Im nächsten Schritt wird der Extrakt in einen Sedimenter gepumpt. Nach 20-30 min Absetzzeit können einzelne, klar getrennte Fraktionen abgezogen werden. Im oberen Teil finden sich dabei Methylester und Pflanzenöl, darunter ein Gemisch von Methanol, Seife, Glyzerin und Wasser und als letzte Phase schließlich ein Sumpf, welcher im Wesentlichen aus neutralisiertem Rotschlamm besteht. Die flüssigen Fraktionen werden abgezogen, wobei in folgenden Reaktoreinheiten jeweils Hexan bzw. nicht abreagiertes Methanol unter Vakuum abdestilliert und anschließend wieder in den Kreislauf zurückgeführt werden.

Das überschüssige Pflanzenöl und die entstandenen Methylester werden abschließend feingefiltert und stehen als wertvolle Produkte zur Verfügung. Die Ausbeute an Methylester liegt dabei üblicherweise bei mindestens 40 %. Seife, Glyzerin und Wasser können in einem weiteren Reaktionsschritt umgewandelt werden, wobei Soda, Wasser und Kohlenwasserstoffe entstehen, die als zusätzliche Wertstoffe aufgefangen und weiterverwendet werden können.

Der Sumpf wird ebenfalls abgepumpt, gegebenenfalls zur Erhöhung der Kraftstoffausbeute zwei- oder mehrmals mit Hexan extrahiert und sedimentiert. Nach einem groben Abfiltrieren über einen Trommelfilter wird der entstandene Filterkuchen über einen Schraubentunnel per Förderschnecke zu einer Pelletpresse gebracht. In der Pelletpresse entsteht neben den Pellets zusätzlich ausgepresste Flüssigkeit, die praktisch alle Bestandteile aus den vorherigen Schritten enthält. Diese wird gesammelt und periodisch zusammen mit neuem bzw. zuvor rückgewonnenem Pflanzenöl zur vollständigen Verwertung wieder in den Reaktor zurückgepumpt.

Die Pellets werden in einen für diese Form des Energieträgers optimierten Ofen bzw. Dampferzeuger gebracht, in welchem dann die organischen Bestandteile zu CO₂ und Wasser verbrannt werden. Wegen des hohen Energiegehalts des Pflanzenöls können dabei entsprechend hohe Energiemengen freigesetzt werden. Der erzeugte Wasserdampf wird vorteilhaft als Heizmedium - beispielsweise zum Vorwärmen des Pflanzenöls - genutzt, etwaige Überschüsse können verstromt werden. Zusätzlich wird Energie aus der Oxidation von im Rotschlamm enthaltenen Eisenhydroxiden zu Hämatit frei. Die zurückbleibende Asche, welche neutral ist oder auf einen leicht basischen pH-Wert eingestellt werden kann, wird abschließend deponiert oder kann nach Reduktion und Abtrennung des Eisenminerals als Füllstoff, Baustoff, Bodenverbesserer oder Mineraldünger verwendet werden.

## Patentansprüche

1. Verfahren zur Umesterung mindestens einer, mindestens eine Estergruppe umfassenden Komponente mit mindestens einer, mindestens eine Hydroxygruppe umfassenden Komponente,
**dadurch gekennzeichnet,**
**dass** dem Verfahren Rotschlamm, welcher durch das zur Aluminiumherstellung verwendete Bayer-Verfahren hergestellt wird, als reaktionsfördernde Komponente zugesetzt wird.

2. Verfahren nach Anspruch 1,
durch folgende Schritte **gekennzeichnet**:
a) Vermischen der mindestens eine Estergruppe umfassenden Komponente, der mindestens eine Hydroxygruppe umfassenden Komponente und dem Rotschlamm;
b) Erwärmen und/oder Durchmischen des erzeugten Gemischs für die Dauer eines vorbestimmten Zeitintervalls; und
c) Abtrennen wenigstens einer ersten flüssigen Phase von wenigstens einer zweiten Phase, wobei die flüssige Phase wenigstens einen Carbonsäurealkylester und die zweite Phase wenigstens dealkalisierten Rotschlamm umfasst.

3. Verfahren nach Anspruch 1 oder 2,
**dadurch gekennzeichnet,**
**dass** die mindestens eine Estergruppe umfassende Komponente ein Pflanzenöl, insbesondere Rapsöl und/oder Palmöl und/oder Sojaöl umfasst.

4. Verfahren nach Anspruch 1 oder 2,
**dadurch gekennzeichnet,**
**dass** die mindestens eine Hydroxygruppe umfassende Komponente wenigstens eine Verbindung aus der Gruppe der C₁- bis C₄-Monoalkohole umfasst.

5. Verfahren nach einem der vorhergehenden Ansprüche,
**dadurch gekennzeichnet,**
**dass** wenigstens eine Komponente in einem Lösungsmittel gelöst ist und/oder selbst als Lösungsmittel fungiert.

6. Verfahren nach einem der Ansprüche 2 bis 5,
**dadurch gekennzeichnet,**
**dass** in Schritt a) zunächst die mindestens eine Hydroxygruppe umfassende Komponente mit dem Rotschlamm vermischt wird und anschließend eine Zumischung der mindestens eine Estergruppe umfassenden Komponente erfolgt.

7. Verfahren nach einem der Ansprüche 2 bis 6,
**dadurch gekennzeichnet,**
**dass** eine Temperatur wenigstens einer der Komponenten vor ihrer Zumischung auf einen vorbestimmten Temperaturwert eingestellt wird.

8. Verfahren nach einem der Ansprüche 2 bis 7,
**dadurch gekennzeichnet,**
**dass** das Gemisch in Schritt b) für die Dauer eines etwa 60-minütigen Zeitintervalls auf etwa 60°C erwärmt und/oder zumindest zeitweise durchmischt wird.

9. Verfahren nach einem der Ansprüche 2 bis 8,
**dadurch gekennzeichnet,**
**dass** nach Schritt a), insbesondere während Schritt b) eine vierte Komponente aufgefangen wird, welche wenigstens eine bei Raumtemperatur und Normaldruck gasförmige Komponente umfasst.

10. Verfahren nach einem der Ansprüche 2 bis 9,
**dadurch gekennzeichnet,**
**dass** das Verfahren nach Schritt b) einen zusätzlichen Schritt d) umfasst, welcher das gemeinsame Abtrennen wenigstens einer fünften und einer sechsten Komponente aus dem Gemisch umfasst, wobei die fünfte Komponente wenigstens eine Verbindung aus der Gruppe der C₁- bis C₄-Monoalkohole und die sechste Komponente wenigstens Wasser umfasst.

11. Verfahren nach Anspruch 10,
**dadurch gekennzeichnet,**
**dass** Schritt d) wenigstens eine Variation von Temperatur- und/oder Druckbedingungen umfasst.

12. Verfahren nach Anspruch 11,
**dadurch gekennzeichnet,**
**dass** die Variation der Temperaturbedingungen eine Regulierung des Temperaturwerts auf etwa 98°C umfasst.

13. Verfahren nach Anspruch 11,
**dadurch gekennzeichnet,**
**dass** die Variation der Temperaturbedingungen eine Regulierung des Temperaturwerts auf etwa 80°C und die Variation der Druckbedingungen eine Regulierung des Druckwerts auf einen gegenüber dem Normaldruck stark verminderten Wert, vorzugsweise unter 250 hPa umfasst.

14. Verfahren nach einem der Ansprüche 10 bis 13,
**dadurch gekennzeichnet,**
**dass** das Verfahren nach Schritt d) einen zusätzlichen Schritt e) umfasst, in welchem die in Schritt d) gemeinsam abgetrennte fünfte und sechste Komponente aufgetrennt wird.

15. Verfahren nach Anspruch 14,
**dadurch gekennzeichnet,**
**dass** Schritt e) wenigstens ein Verfahren zur Trennung homogener Gemische, insbesondere ein Extraktivdestillationsverfahren umfasst.

16. Verfahren nach Anspruch 15,
**dadurch gekennzeichnet,**
**dass** das Verfahren zur Trennung homogener Gemische ein Schleppmittel, insbesondere Glycerin und/oder Paraffinöl und/oder Ethandiol umfasst.

17. Verfahren nach einem der Ansprüche 2 bis 16,
**dadurch gekennzeichnet,**
**dass** Schritt c) wenigstens ein Destillations- und/oder Filterungs- und/oder Sedimentations- und/oder Dekantierverfahren umfasst.

18. Verfahren nach Anspruch 17,
**dadurch gekennzeichnet,**
**dass** wenigstens ein Sedimentationsverfahren ein Abtrennen von Rotschlamm-Sedimenten umfasst.

19. Verfahren nach Anspruch 18,
**dadurch gekennzeichnet,**
**dass** wenigstens ein Filterungsverfahren einen Filterungsschritt mittels Rotschlamm-Sedimenten umfasst.

20. Verfahren nach Anspruch 17,
**dadurch gekennzeichnet,**
**dass** wenigstens ein Filterungsverfahren einen Filterungsschritt mittels eines Vakuumfilters umfasst.

21. Verfahren nach Anspruch 20,
**dadurch gekennzeichnet,**
**dass** der Vakuumfilter mehrstufig ausgebildet ist.

22. Verfahren nach einem der Ansprüche 19 bis 21,
**dadurch gekennzeichnet,**
**dass** zumindest ein Teil eines durch den Filterungsschritt erzeugten Filterkuchens thermisch verwertet, insbesondere verbrannt wird.

23. Verfahren nach Anspruch 22,
**dadurch gekennzeichnet,**
**dass** dem Filterkuchen vor dem thermischen Verwerten Kunststoff- und/oder Holz- und/oder biologische Abfälle zugemischt werden.

24. Verfahren nach Anspruch 22 oder 23,
**dadurch gekennzeichnet,**
**dass** beim thermischen Verwerten entstehende thermische Energie wenigstens einem der Verfahrensschritte a) bis c) und/oder gegebenenfalls d) und/oder e) zugeführt wird.

25. Verfahren nach einem der Ansprüche 2 bis 24,
**dadurch gekennzeichnet,**
**dass** nach Schritt c) ein zusätzlicher Schritt f) ein Abtrennen mindestens einer, mindestens Glycerin umfassenden Komponente umfasst.

26. Verfahren nach Anspruch 25,
**dadurch gekennzeichnet,**
**dass** nach Schritt f) in einem weiteren Schritt g) die mindestens eine, mindestens Glycerin umfassende Komponente in wenigstens zwei Qualitätsstufen aufgetrennt wird.

27. Verfahren nach einem der Ansprüche 2 bis 26,
**dadurch gekennzeichnet,**
**dass** nach Schritt c) in einem weiteren Schritt h) mindestens ein Natriumionen enthaltendes Salz mindestens einer Carbonsäure aus wenigstens einer abgetrennten Phase abgetrennt wird.

28. Verwendung des in Schritt h) gemäß Anspruch 27 abgetrennten mindestens einen Natriumionen enthaltenden Salzes mindestens einer Carbonsäure (Seife) als Pflanzenbehandlungsmittel, insbesondere zum Behandeln von Schädlingsbefall.

29. Verwendung nach Anspruch 28, wobei das mindestens eine Natriumionen enthaltende Salz mindestens einer Carbonsäure mit mindestens einem Lösungsmittel, insbesondere Wasser verdünnt ist.

30. Verwendung des in Schritt h) gemäß Anspruch 27 abgetrennten mindestens einen Natriumionen enthaltenden Salzes mindestens einer Carbonsäure als Detergens, insbesondere in Reinigungs- und/oder Waschmitteln.

31. Verwendung des in Schritt c) gemäß Anspruch 2 abgetrennten, dealkalisierten Rotschlamms als Eisen liefernder Bestandteil eines insbesondere in der Landwirtschaft verwendbaren Eisendüngers.

32. Verwendung nach Anspruch 31,**dadurch gekennzeichnet, dass** der Eisendünger zusätzlich zumindest Kalkstein umfasst.

## Claims

1. Method for transesterification of at least one component including at least one ester group with at least one component including at least one hydroxyl group,
**characterized in that**
red mud produced by the Bayer process used for aluminum production is added to the method as a reaction-promoting component.

2. Method according to claim 1,
**characterized by** the following steps:
a) mixing the component including at least one ester group, the component including at least one hydroxyl group, and the red mud;
b) heating and/or mixing the generated mixture for the duration of a predetermined time interval; and
c) separating at least one first liquid phase from at least one second phase,
wherein the liquid phase includes at least one carboxylic acid alkyl ester and the second phase includes at least dealkalized red mud.

3. Method according to claim 1 or 2,
**characterized in that**
the component including at least one ester group comprises a vegetable oil, especially rape oil and/or palm oil and/or soya oil.

4. Method according to claim 1 or 2,
**characterized in that**
the component including at least one hydroxyl group comprises at least one compound of the group of the C₁ to C₄ monoalcohols.

5. Method according to anyone of the preceding claims,
**characterized in that**
at least one component is dissolved in a solvent and/or functions itself as a solvent.

6. Method according to any one of claims 2 to 5,
**characterized in that**
in step a), first the component including at least one hydroxyl group is mixed with the red mud and subsequently an admixture of the component including at least one ester group is effected.

7. Method according to any one of claims 2 to 6,
**characterized in that**
a temperature of at least one of the components is adjusted to a predetermined temperature value before admixture thereof.

8. Method according to any one of claims 2 to 7,
**characterized in that**
the mixture is heated to about 60 °C in step b) for the duration of a time interval of about 60 minutes and/or at least partially mixed.

9. Method according to any one of claims 2 to 8,
**characterized in that**
after step a), especially during step b), a fourth component is collected, which includes at least one component gaseous at room temperature and normal pressure.

10. Method according to any one of claims 2 to 9,
**characterized in that**
the method includes an additional step d) after step b), which includes the collective separation of at least one fifth and one sixth component from the mixture, wherein the fifth component includes at least one compound of the group of the C₁ to C₄ monoalcohols and the sixth component includes at least water.

11. Method according to claim 10,
**characterized in that**
step d) includes at least one variation of temperature and/or pressure conditions.

12. Method according to claim 11,
**characterized in that**
the variation of the temperature conditions includes regulation of the temperature value to about 98 °C.

13. Method according to claim 11,
**characterized in that**
the variation of the temperature conditions includes regulation of the temperature value to about 80 °C, and the variation of the pressure conditions includes regulation of the pressure value to a value greatly decreased with respect to normal pressure, preferably below 250 hPa.

14. Method according to any one of claims 10 to 13,
**characterized in that**
the method includes an additional step e) after step d), in which the fifth and sixth components collectively separated in step d) are separated.

15. Method according to claim 14,
**characterized in that**
step e) includes at least one process for separating homogeneous mixtures, especially an extractive distillation process.

16. Method according to claim 15,
**characterized in that**
the process for separating homogeneous mixtures includes an entrainer, especially glycerin and/or paraffin oil and/or ethane diol.

17. Method according to any one of claims 2 to 16,
**characterized in that**
step c) includes at least one distillation and/or filtering and/or sedimentation and/or decantation process.

18. Method according to claim 17,
**characterized in that**
at least one sedimentation process includes separation of red mud sediments.

19. Method according to claim 18,
**characterized in that**
at least one filtering process includes a filtering step by means of red mud sediments.

20. Method according to claim 17,
**characterized in that**
at least one filtering process includes a filtering step by means of a vacuum filter.

21. Method according to claim 20,
**characterized in that**
the vacuum filter is formed in multiple stages.

22. Method according to any one of claims 19 to 21,
**characterized in that**
at least a part of a filter cake produced by the filtering step is thermally utilized, especially combusted.

23. Method according to claim 22,
**characterized in that**
plastic and/or wood and/or biological waste is admixed with the filter cake before thermal utilization.

24. Method according to claim 22 or 23,
**characterized in that**
thermal energy arising in thermal utilization is supplied to at least one of the method steps a) to c) and/or optionally d) and/or e).

25. Method according to any one of claims 2 to 24,
**characterized in that**
an additional step f) after step c) includes separation of at least one component including at least glycerin.

26. Method according to claim 25,
**characterized in that**
after step f), in a further step g), the at least one component including at least glycerin is separated into at least two degrees of quality.

27. Method according to any one of claims 2 to 26,
**characterized in that**
after step c), in a further step h), at least one salt of at least one carboxylic acid containing sodium ions is separated from at least one separated phase.

28. Use of the at least one salt of at least one carboxylic acid (soap) containing sodium ions, separated in step h) according to claim 27, as a plant treating agent, especially for treating pest infestation.

29. Use according to claim 28, wherein the at least one salt of at least one carboxylic acid containing sodium ions is diluted with at least one solvent, especially water.

30. Use of the at least one salt of at least one carboxylic acid containing sodium ions separated in step h) according to claim 27 as a detergent, especially in cleaning and/or washing agents.

31. Use of the dealkalized red mud separated in step c) according to claim 2 as an iron-contributing component of an iron fertilizer especially usable in the agriculture.

32. Use according to claim 31, **characterized in that** the iron fertilizer additionally includes at least limestone.

## Revendications

1. Procédé de transesterification d'au moins un composant comprenant au moins un groupe d'esters avec au moins un composant comprenant au moins un groupe hydroxy **caractérisé en ce que** le procédé prévoit l'ajout de boue rouge en tant que composant promouvant la réaction qui résulte du procédé Bayer pour la production d'aluminium.

2. Procédé selon la revendication 1, **caractérisé par** les étapes suivantes :
a) Mélanger le composant comprenant au moins un groupe d'esters avec le composant comprenant au moins un groupe hydroxy et avec la boue rouge ;
b) Réchauffer et/ou mélanger le mélange en résultant pendant la durée d'un intervalle déterminé et
c) Séparer au moins une première phase liquide d'au moins une deuxième phase, la phase liquide comprenant au moins un ester alkylique d'acide carboxylique et la deuxième phase comprenant au moins de la boue rouge désalcalysée.

3. Procédé selon la revendication 1 ou 2, **caractérisé en ce que** le composant comprenant au moins un groupe d'esters comprend une huile végétale et notamment de l'huile de colza et/ou de l'huile de palmier et/ou de l'huile de soja.

4. Procédé selon la revendication 1 ou 2, **caractérisé en ce que** le composant comprenant au moins un groupe hydroxy comprend au moins un composé du groupe des mono-alcools C₁ à C₄.

5. Procédé selon l'une des revendications susmentionnées, **caractérisé en ce qu'**au moins un composant est dissout dans un solvant et/ou agit lui-même en tant que solvant.

6. Procédé selon l'une des revendications 2 à 5, **caractérisé en ce que**, dans l'étape a), le composant comprenant au moins un groupe hydroxy est mélangé avec la boue rouge et ensuite avec le composant comprenant au moins un groupe d'esters.

7. Procédé selon l'une des revendications 2 à 6, **caractérisé en ce que** la température d'au moins un des composants est réglée sur une température définie avant de l'ajouter.

8. Procédé selon l'une des revendications 2 à 7, **caractérisé en ce que** le mélange est réchauffé à environ 60°C et/ou au moins mélangé temporairement pendant un intervalle d'un durée d'environ 60 minutes dans le cadre de l'étape b).

9. Procédé selon l'une des revendications 2 à 8, **caractérisé en ce que**, après l'étape a) et notamment pendant l'étape b), un quatrième composant est collecté qui comprend au moins un composant qui est gazeux à température ambiante et à pression atmosphérique.

10. Procédé selon l'une des revendications 2 à 9, **caractérisé en ce que** le procédé comprend une étape supplémentaire d) après l'étape b) qui englobe la séparation d'au moins un cinquième et d'un sixième composant à partir du mélange, le cinquième composant comprenant au moins un composé du groupe des mono-alcools C₁ à C₄ et le sixième composant comprenant au moins de l'eau.

11. Procédé selon la revendication 10, **caractérisé en ce que** l'étape d) prévoit au moins une variation de la température et/ou de la pression.

12. Procédé selon la revendication 11, **caractérisé en ce que** la variation de la température prévoit le réglage de la température sur environ 98°C.

13. Procédé selon la revendication 11, **caractérisé en ce que** la variation de la température prévoit le réglage de la température à environ 80°C et que la variation de la pression prévoit le réglage de la pression à une pression fortement réduite par rapport à la pression atmosphérique et de préférence inférieure à 250 hPa.

14. Procédé selon l'une des revendications 10 à 13, **caractérisé en ce que** le procédé englobe une étape supplémentaire e) après l'étape d) où le cinquième et le sixième composant conjointement séparés dans l'étape d) sont séparés l'un de l'autre.

15. Procédé selon la revendication 14 **caractérisé en ce que** l'étape e) prévoit au moins un procédé pour la séparation de mélanges homogènes et notamment un procédé de distillation extractive.

16. Procédé selon la revendication 15 **caractérisé en ce que** le procédé de séparation de mélanges homogènes prévoit l'utilisation d'un agent d'entraînement tel que notamment la glycérine et/ou l'huile de paraffine et/ou l'éthane-diol.

17. Procédé selon l'une des revendications 2 à 16 **caractérisé en ce que** l'étape c) prévoit au moins un procédé de distillation et/ou de filtration et/ou de sédimentation et/ou de décantation.

18. Procédé selon la revendication 17 **caractérisé en ce qu'**au moins un procédé de sédimentation prévoit la séparation de sédiments de boue rouge.

19. Procédé selon la revendication 18 **caractérisé en ce qu'**au moins un procédé de filtration prévoit une étape de filtration par sédiments de boue rouge.

20. Procédé selon la revendication 17 **caractérisé en ce qu'**au moins un procédé de filtration prévoit une étape de filtration par filtre à vide.

21. Procédé selon la revendication 20 **caractérisé en ce que** le filtre à vide comprend plusieurs étages.

22. Procédé selon l'une des revendications 19 à 21 **caractérisé en ce qu'**au moins une partie du gâteau de filtration crée par l'étape de filtration est exploitée thermiquement et notamment par combustion.

23. Procédé selon la revendication 22 **caractérisé en ce que** des déchets de plastique et/ou de bois et ou des déchets biologiques sont ajoutés au gâteau de filtration avant son exploitation thermique.

24. Procédé selon la revendication 22 ou 23 **caractérisé en ce que** l'énergie thermique créée lors de l'exploitation thermique est utilisée pour au moins une des étapes du procédé a) à c) et/ou, le cas échéant, d) et/ou e).

25. Procédé selon l'une des revendications 2 à 24 **caractérisé en ce qu'**une étape supplémentaire f) après l'étape c) prévoit la séparation d'au moins un composant comprenant au moins de la glycérine.

26. Procédé selon la revendication 25 **caractérisé en ce qu'**une étape supplémentaire g) après l'étape f) prévoit la séparation d'au moins un composant comprenant au moins de la glycérine, dans au moins deux niveaux de qualité.

27. Procédé selon l'une des revendications 2 à 26 **caractérisé en ce qu'**une étape supplémentaire h) après l'étape c) prévoit la séparation d'au moins un sel contenant des ions de natrium d'au moins un acide carboxylique en tant qu'agent de traitement de plantes et notamment pour le traitement anti-parasites.

28. Utilisation du sel contenant des ions de natrium d'au moins un acide carboxylique (savon) et ayant été séparé dans le cadre de l'étape h) selon la revendication 27 en tant qu'agent de traitement de plantes et notamment pour le traitement anti-parasites :

29. Utilisation selon la revendication 28, le sel contenant des ions de natrium d'au moins un acide carboxylique étant dilué avec au moins un solvant et notamment avec de l'eau.

30. Utilisation du sel contenant des ions de natrium d'au moins un acide carboxylique et ayant été séparé dans le cadre de l'étape h) selon la revendication 27 en tant que détergent et notamment dans des produits de nettoyage et/ou la lessive.

31. Utilisation de la boue rouge désalcalisée séparée dans le cadre de l'étape c) selon la revendication 2 en tant que composant fournissant du fer dans un engrais ferreux pouvant être utilisé notamment dans l'agriculture.

32. Utilisation selon la revendication 31 **caractérisée en ce que** l'engrais ferreux comprend en outre au moins de la chaux.
